# FASCICULE DE BREVET EUROPEEN

(11) **EP 2 791 685 B1**
(45) Date de publication et mention de la délivrance du brevet: **24.10.2018**
(21) Numéro de dépôt: 12813912.8
(22) Date de dépôt: 12.12.2012
(51) Int. Cl.: G01N 33/68, A61L 2/00, A61M 1/02

(54) **NOUVELLE UTILISATION D'UN MATÉRIAU CAPABLE DE LIER LA PROTÉINE PRION**
NEUE VERWENDUNG EINES MATERIALS ZUR BINDUNG VON PRIONPROTEIN
NOVEL USE OF A MATERIAL CAPABLE OF BINDING PRION PROTEIN

(30) Priorité: 13.12.2011 FR 1161588
(43) Date de publication de la demande: 22.10.2014
(73) Titulaire: Maco Pharma, 59420 Mouvaux (FR)
(72) Inventeur: COMOY, Emmanuel, F-91190 Saint-Aubin (FR); SUMIAN, Chryslain, F-59960 Neuville en Ferrain (FR); LESCOUTRA, Nathalie, F-78000 Versailles (FR); JAFFRE, Nina, F-75014 Paris (FR); DESLYS, Jean-Philippe, F-78150 Le Chesnay (FR)
(74) Mandataire: Sayettat, Julien Christian
(86) Numéro de dépôt international: PCT/FR2012/052908
(87) Numéro de publication internationale: WO 2013/088070

(56) Documents cités:
- WO-A2-2004/090102
- GREGORI L ET AL: "Reduction in infectivity of endogenous transmissible spongiform encephalopathies present in blood by adsorption to selective affinity resins", THE LANCET, LANCET LIMITED. LONDON, GB, vol. 368, no. 9554, 23 décembre 2006 (2006-12-23), pages 2226-2230, XP027947575, ISSN: 0140-6736 [extrait le 2006-12-23] cité dans la demande
- MIKIHIRO YUNOKI ET AL: "Infectious prion protein in the filtrate even after 15nm filtration", BIOLOGICALS, vol. 38, no. 2, 1 mars 2010 (2010-03-01), pages 311-313, XP055028613, ISSN: 1045-1056, DOI: 10.1016/j.biologicals.2009.10.018
- PIERLUIGI GAMBETTI ET AL: "A novel human disease with abnormal prion protein sensitive to protease", ANNALS OF NEUROLOGY, vol. 63, no. 6, 1 juin 2008 (2008-06-01), pages 697-708, XP055028596, ISSN: 0364-5134, DOI: 10.1002/ana.21420

## Description

L'invention concerne une nouvelle utilisation d'un matériau capable de se lier à la protéine prion pour éliminer, dans un produit biologique, l'agent étiologique induisant une maladie neurodégénérative autre que les maladies de Creutzfeldt-Jakob.

L'invention s'applique de façon générale au traitement de produits biologiques, tels que le sang ou les composants sanguins, les milieux cellulaires, ou les tissus biologiques. L'invention s'applique notamment à la transfusion de composants sanguins ou des dérives du plasma sanguin.

Le prion est l'agent responsable des encéphalopathies subaiguës spongiformes transmissibles, notamment de la maladie de Creutzfeldt-Jakob (MCJ) chez l'homme. Des études récentes ont montré qu'il existe un risque probable de transmission du prion lors de transfusion sanguine. Ce risque concernant la transmission par le sang d'infections par le prion a été confirmé par l'apparition au Royaume-Uni de quatre cas de vMCJ (variante de la maladie de Creutzfeldt-Jakob) liées à une transfusion. Les périodes d'incubation asymptomatiques dans les maladies à prions peuvent s'étendre sur plusieurs décennies chez les humains, et le syndrome de la maladie typique peut survenir ou pas.

Ainsi, il est apparu nécessaire d'éliminer le prion des produits sanguins destinés à être transfusés. Dans les documents WO-2004/090102 et WO-2006/044459, il est décrit des particules adsorbantes sous forme de résine capable de lier de façon sélective les protéines prion présentes dans le sang. Ces particules adsorbantes sont placées dans des colonnes.

Ces particules sont intégrées dans le filtre commercial P-CAPT™ (Maco Pharma, France), décrit dans le document WO-2007/042644. Elles possèdent une forte affinité pour la protéine prion normale (PrP et PrPc) et pour la protéine prion résistante à la protéinase K (PrPres) associée aux maladies à prion, notamment la vMCJ (Gregori et al., 2006, Transfusion, 46, 1152-1161) et éliminent l'infectiosité endogène du sang dans un modèle expérimental de hamster (Gregori et al., Lancet, 368, 2226-2230).

Dans le document Yunoki et al., 2010, Biologicals, 38, 311-313, il est décrit un procédé pour éliminer l'agent étiologique PrPres de la maladie de scrapie présente dans le sang en utilisant un filtre ayant des pores de 15 nm.

Le document Gambetti et al., 2008. Am J Neurol Assoc, 63(6), 697-708, décrit une maladie neurodégénérative à prions ne présentant pas d'accumulation de PrPres dans le néocortex. La protéine prion anormale induisant la maladie neurodégénérative possède une structure très différente de PrPres.

L'invention repose sur une étude montrant pour la première fois que la transfusion de composants sanguins issus d'animaux atteints de la vMCJ induit une maladie neurologique non encore décrite, dépourvue des caractéristiques classiques des maladies à prions, mais caractérisée par une parésie des membres supérieurs, des lésions centrées sur les cornes antérieures de la moelle épinière, et une absence de spongiose ou d'inflammation. Chez ces animaux atteints, la protéine prion anormale (PrPres) n'a pas été détectée par les tests standards en usage pour le diagnostic du prion humain. Il est à noter que les animaux transfusés avec du sang infectieux traité avec le filtre P-CAPT™ ne développent pas cette maladie.

Il est parfaitement inattendu qu'une nouvelle maladie neurologique soit induite par des produits sanguins ou des dérivés du sang provenant de donneurs en incubation silencieuse ou en phase clinique de la MCJ et ce, sans aucune accumulation de protéine anormale du prion (PrPres), mais il est encore plus inattendu q'un matériau capable de lier la protéine prion puisse protéger de cette maladie alors qu'aucune protéine anormale n'est détectable.

L'invention propose donc une nouvelle utilisation d'un matériau capable de se lier à la protéine prion pour éliminer, dans un produit biologique, l'agent étiologique induisant une maladie neurodégénérative autre que les maladies de Creutzfeldt-Jakob.

D'autres objets et avantages apparaîtront au cours de la description qui suit.

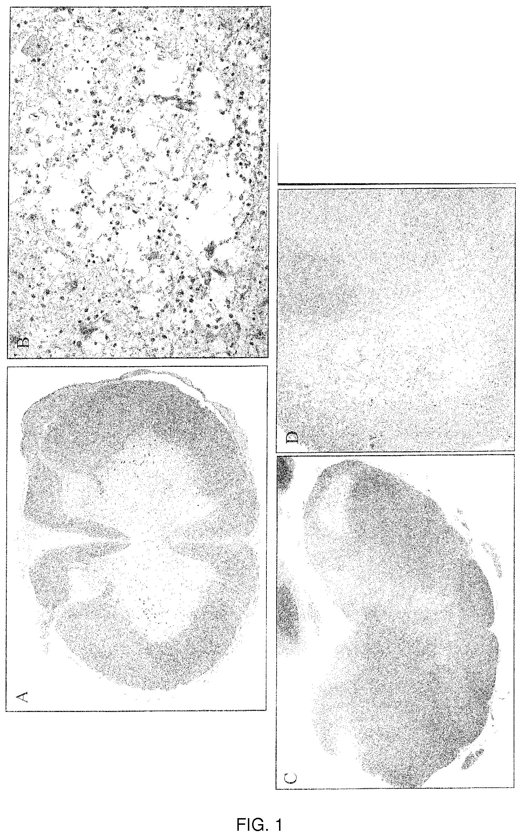

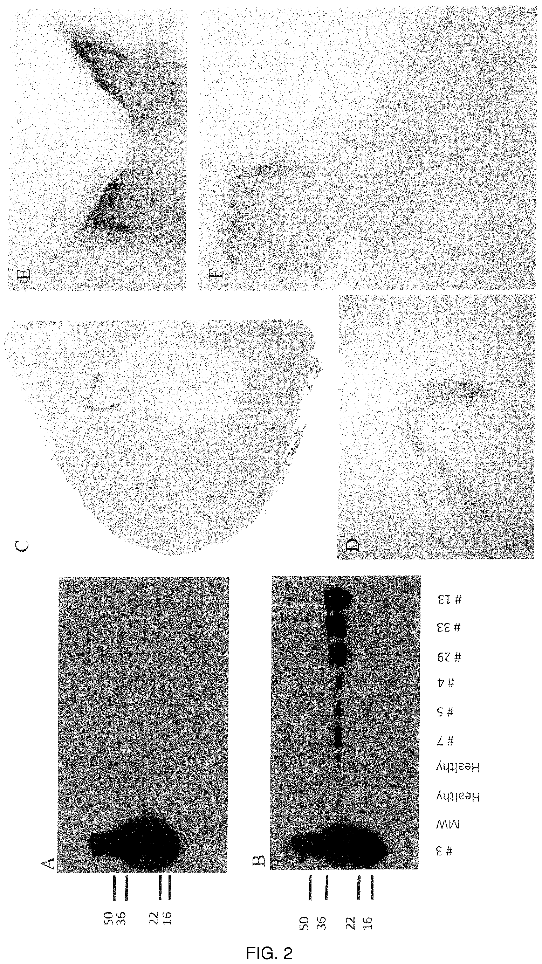

Les figures 1A-1D sont des photos des lésions du SNC dans des infections atypiques de primates. La figue 1A) est le segment C6 de la moelle épinière du primate #13 présentant des lésions nécrotiques des cornes antérieures avec perte de neurones et une dégénérescence des cordons postérieurs (coloration par Luxol, x12.5). La figure 1B est un agrandissement montrant la gliose à la périphérie de la lésion. (coloration par HE, x200). Les figures 2C et 2D représentent le primate #7 avec des lésions nécrotiques bilatérales dans les noyaux spinaux du nerf trijumeau et les pédoncules cérébelleux inférieurs. (coloration H & E, x12.5 et x50).

Les figures 2A-2E illustrent la détection de la PrP dans les infections atypiques chez le primate. Une purification de la PrP est réalisée en présence (figure 2A) ou en l'absence (figure 2B) de protéolyse, puis celle-ci est détectée avec l'anticorps 3F4. Dans la moelle cervicale, seules les cornes postérieures présentent un marquage par l'anticorps 3F4 chez le primate atypique #5 (Figure 2C, x12.5) et #13 (Figure 3D, x100), alors que toute la substance grise est marquée chez les primates #2 (Figure 2E , x50) et #15 (Figure 2F, X50) infectés par vMCJ ou ESB et présentant des signes cliniques et de lésions attendus.

L'invention concerne une nouvelle utilisation d'un matériau capable de lier la protéine prion pour éliminer, dans un produit biologique, l'agent étiologique induisant une maladie neurodégénérative autre que les maladies de Creutzfeldt-Jakob, de sorte à éviter la transmission de cette maladie au receveur dudit produit biologique.

Selon une réalisation particulière, le matériau capable de lier la protéine prion est un matériau tel que décrit dans les documents WO-2004/090102 et WO-2006/044459.

Notamment, le matériau comprend une matrice liée à un groupement fonctionnel. Le groupement fonctionnel est un groupement hydrophile, hydrophobe ou amphiphile.

Par exemple, le groupement fonctionnel est une amine primaire, notamment le groupe -OCH2-CHOH-CH2-NH2. Dans un autre exemple, le groupement fonctionnel est un groupe phényle, n-butyle, 2-(diméthylammonio)éthyle, 2-(triméthylammonio)éthyle, diméthylaminoéthyle ou triméthylaminoéthyle.

La matrice est une matrice minérale ou organique. Lorsque la matrice est minérale, elle comprend de la silice ou de l'alumine. Lorsque la matrice est organique, elle comprend notamment une matrice polymère poreuse telle qu'une matrice réalisée en polyméthacrylate ou en méthacrylate, une matrice Fractogel™, Toyopearl™ or TSK-GEL™.

Les matrices Fractogel™, Toyopearl™ or TSK-GEL™ sont des matrices commercialisées par Tosoh Bioscience.

En particulier, la matrice est une matrice Toyopearl™ amino 650, tel que la matrice Toyopearl™ amino 650 U ou Amino 650 M.

Le matériau capable de lier la protéine prion est utilisé sec ou humide. Par exemple, le matériau est mouillé avant utilisation avec une solution additive de type SAGM (Sodium Adénine Glucose Mannitol).

Dans un exemple particulier, le matériau capable de lier la protéine prion est une résine de type Prioclear™ commercialisée par Prometic Bioscience.

Par matériau capable de lier la protéine prion, on entend un matériau capable de lier la protéine prion normale (PrP ou PrPc) ou la protéine prion anormale, c'est-à-dire la forme infectieuse de la protéine prion caractérisée par sa résistance à la protéinase K (PrPres).

Ce matériau est capable d'éliminer d'un produit biologique l'agent étiologique induisant une nouvelle maladie neurodégénérative, notamment lors de la transfusion ou l'injection à un patient dudit produit biologique.

En particulier, le produit biologique est issu d'un individu infecté par une souche de prion, notamment l'agent étiologique responsable des maladies de Creutzfeldt Jakob.

Le but de la nouvelle utilisation du matériau capable de lier la protéine prion est bien de protéger les patients recevant du sang ou des dérivés sanguins provenant de donneurs potentiellement exposés à l'agent de l'ESB ou de la vMCJ. En effet, l'étude décrite ci-dessous montre qu'il est désormais possible d'imaginer que des patients en incubation silencieuse de formes classiques de la MCJ voire des porteurs sains pourraient donner leur sang et transmettre ces nouveaux agents infectieux qui restent à caractériser plus directement.

Ce produit ainsi traité puis injecté ou transfusé à un patient n'engendre pas l'apparition de cette nouvelle maladie neurodégénérative.

Un produit biologique comprend par exemple le sang, un composant sanguin tel que du sérum, du plasma, des plaquettes, des globules blancs, des globules rouges, un buffy coat, un lysat plaquettaire, des plaquettes, ou la moelle osseuse. Dans ce cas, le produit biologique est déleucocyté ou non, par exemple par passage au travers d'un filtre à déleucocyter.

Dans un autre exemple, le produit biologique comprend des préparations de protéines issues du sang ou du plasma sanguin telles que des préparations de facteurs de coagulation, d'enzymes, d'albumine, de plasminogène, et d'immunoglobulines. Notamment, les préparations de facteurs de coagulation comprennent les préparations de fibrinogène, de facteur XIII, de thrombine, de facteur VIII, du facteur de von Willebrand, de protéine C, ou de protéine C activés.

Selon une réalisation particulière, le matériau capable de se lier à la protéine prion est incorporé dans une colonne chromatographique.

Selon une autre réalisation, le matériau est placé dans une unité de filtration comprenant une ou plusieurs couches d'une matière poreuse, notamment réalisées en non-tissé.

Le non-tissé est par exemple réalisé dans un matériau polymère tel que le polyéthylène téréphtalate, le polybutylène téréphtalate ou le polypropylène.

Par exemple, le matériau est disposé dans une unité de filtration telle que décrite dans le document WO-2007/042644. Dans une telle unité de filtration, le matériau est mis en sandwich entre deux couches de non-tissé.

Par exemple, ce matériau est présent dans le filtre P-CAPT™ (Maco Pharma, France) disponible dans le commerce.

Selon l'invention, le matériau est utilisé pour éliminer d'un produit biologique l'agent étiologique induisant une maladie neurodégénérative autre que les maladies de Creutzfeldt Jakob, c'est-à-dire différente de son expression de la forme attendue. Les maladies de Creutzfeldt-Jakob incluent la forme normale de la maladie de Creutzfeldt-Jakob, sa variante (vMCJ) ou sa forme sporadique (sMCJ).

Cette maladie neurodégénérative autre que la MCJ se caractérise notamment par une dégénérescence de la moelle épinière au niveau des cornes antérieures.

Le produit biologique est notamment issu d'individus infectés par une souche humaine de prion tel que la vMCJ.

En effet, l'invention se fonde sur la découverte que du sang d'un donneur infecté par la vMCJ ou l'encéphalopathie spongiforme bovine (ESB) peut induire une maladie neurodégénérative jusqu'à ce jour non décrite. La transmission de cette maladie est évitée lorsque le produit biologique est filtré au travers d'un filtre P-CAPT™ (Maco Pharma, France). C'est justement cette observation qui amène à penser que cette nouvelle maladie appartient en fait à la classe des maladies à prions.

L'agent étiologique de cette maladie neurodégénérative n'est pas la protéine prion anormale sous sa forme résistante à la protéolyse (PrPres). En toute hypothèse, l'agent étiologique serait une protéine prion dans une forme atypique non encore connue.

Le but de la nouvelle utilisation de ce matériau capable de lier la protéine prion est d'éviter la transmission de cette nouvelle maladie neurodégénérative soit lors de la transfusion de produits sanguins labiles (sang total, plasma et cellules sanguines) notamment déleucocytés, soit lors d'injection de produits dérivés du sang, ceux notamment obtenus par fractionnement du plasma sanguin.

Chez le macaque cynomolgus, la pathologie observée correspond à une neuropathie non inflammatoire affectant préférentiellement mais non exclusivement la moelle épinière sans atteinte apparente des autres organes.

D'un point de vue clinique, l'évolution est longue (plusieurs mois) et commence par une imprécision des gestes fins des membres supérieurs, interprétés par le sujet comme des troubles de la vision. Une ataxie non cérébelleuse apparaît au niveau des membres inférieurs, et les membres supérieurs présentent une parésie flasque principalement proximale. Les membres supérieurs sont cependant mobilisables. Une atteinte des nerfs crâniaux est suspectée, en présence de bâillements répétés et des mouvements anormaux de la langue. Des troubles de la sensibilité proprioceptive et nociceptive sont observés, principalement au niveau des membres inférieurs et au niveau de la ceinture scapulaire. Des troubles du comportement sont observés de façon inconsistante. Les tremblements sont inconstants, en revanche des trémulations importantes sont observées post mortem.

D'un point de vue lésionnel, les muscles de la ceinture scapulaire, principalement les muscles trapèze, sont atrophiés. Les lésions médullaires sont localisées au niveau des cornes antérieures de la substance grise, et correspondent d'une part à une nécrose tissulaire avec infiltration macrophagique et astrocytaire en l'absence de lymphocyte (les neurones sont en partie préservés) et à une démyélinisation des cordons postérieurs d'autre part (principalement le faisceau gracile). Les lésions de la substance grise sont principalement observées au niveau de la moelle cervicale basse, mais peuvent remonter jusqu'aux cervicales hautes et descendre jusqu'aux dorsales moyennes.

Des lésions identiques de nécrose sont observées de façon bilatérale au niveau des noyaux spinaux des nerfs trijumeaux, pouvant s'étendre au niveau des pédoncules cérébelleux. Les bandelettes optiques présentent également une démyélinisation. Les autres zones du cerveau présentent des lésions neuronales inconstantes.

D'un point de vue biochimique, il n'y a pas d'accumulation de PrP résistante à la protéolyse (PrPres) détectable dans le SNC des individus atteints avec les techniques classiques. En revanche, une accumulation de PrP non résistante à la protéolyse est observable au niveau de la substance gélatineuse des cornes postérieures de la moelle épinière.

Il est envisageable que l'agent étiologique responsable du tableau décrit ci-dessus chez le primate induise une pathologie différente dans une autre espèce, y compris l'homme.

### Exemple

### Contexte

Nous présentons les résultats d'une nouvelle série d'expériences pour évaluer le risque de transmission sanguine d'un agent pathogène non décrit à ce jour dans un modèle primate non humain et validé.

### Méthodes

Des macaques cynomolgus ont été inoculés avec des échantillons de cerveau ou de sang provenant de patients atteints de la vMCJ ou de macaques infectés par la vMCJ ou l'ESB. Les résultats neuropathologiques et biochimiques ont été obtenues en utilisant les méthodes actuelles utilisées pour des patients humains.

### Résultats

La transfusion de sang total à partir d'un primate infecté à la vMCJ transmet la maladie avec le schéma classique attendu. En parallèle, plusieurs primates exposés à des dérivés de sang d'humain ou de macaque présentaient une maladie neurologique jusque-là non décrite ayant pour caractéristiques une parésie des membres supérieurs, des lésions centrées sur les cornes antérieures de la moelle épinière, une absence de spongiose ou d'inflammation. Cette maladie est dépourvue des caractéristiques classiques de la maladie à prions, c'est-à-dire une spongiose, des lésions neuronales et une réaction astrogliale dans l'ensemble du cerveau, principalement mais non exclusivement dans le tronc cérébral, le cervelet et le cortex occipital, et une accumulation de PrPres avec un poids moléculaire de 20 kDa pour sa forme non glycosylée après protéolyse. Chez les animaux présentant cette nouvelle forme de maladie neurologique, la protéine prion anormale (PrPres) n'a pas été détectée par les tests standards en usage pour le diagnostic du prion humain, mais des quantités plus élevées de PrP sensible à la protéase ont été détectées dans la moelle épinière, en comparaison avec les animaux témoins qui ont présentés une forme typique vMCJ ou ESB. Aucune autre cause n'a été trouvée par une recherche exhaustive des étiologies d'origine métabolique, endocrinienne, toxique, nutritionnelle et infectieuse, y compris par une recherche de génotypes pathogènes («séquençage en profondeur»). Par ailleurs, les animaux transfusés avec du sang traité avec le filtre P-CAPT™ (Maco Pharma, France) ne développent pas la maladie.

### Interprétation

Nous décrivons un nouveau syndrome neurologique chez des macaques exposés à divers prions par inoculation ou transfusion. Les infections humaines, si elles se produisaient, ne seraient pas reconnues par les investigations diagnostiques permettant de détecter une MCJ, notamment la vMCJ ou la sMCJ.

Les consommateurs britanniques ont été largement exposés à l'agent prion de l'ESB (encéphalopathie spongiforme bovine) puisque plus de 2 millions de bovins diagnostiqués contaminés seraient entrés dans la chaîne alimentaire humaine (Smith et Bradley, 2003, Br Med Bu1166, 185-198). L'épidémie d'ESB a culminé il y a plusieurs années, et a été en déclin depuis lors, avec seulement quelques cas diagnostiqués en 2011. La transmission primaire orale aux humains a été limitée à 176 cas de variant de la maladie de Creutzfeldt-Jakob (vMCJ), avec une l'incidence annuelle actuelle proche de zéro. Cependant, le risque de transmission secondaire dû aux patients en phase d'incubation de la maladie continue, et pourrait s'étendre sur des décennies, comme observé avec la maladie de Kuru (Collinge et al., 2006, Lancet 367, 2068-2074). Quatre infections associées à la transfusion de globules rouges non déleucocytés ont été rapportées au Royaume-Uni depuis 2003 (Turner et Ludlam, 2009, Br J Haematol 144, 14-23).

En l'absence d'un test sanguin de diagnostic fiable pour dépister les donneurs, des mesures de protection ont été instituées, basées sur des données épidémiologiques et des résultats expérimentaux obtenus avec des modèles de rongeurs et de moutons. Elles reposent principalement sur : i) l'exclusion de donneurs, ii) la réduction des leucocytes qui contiennent la majeure partie de l'infectiosité des prions du sang (Vamvakas, 2011, Transfus Med Rev 25, 133-144), et iii) des techniques de filtration par exclusion de taille (Burnouf et Padilla, 2006, Transfus Clin Biol 13, 320-328) pour des dérivés du plasma.

Pour explorer davantage le risque lié au sang et aux produits sanguins, nous avons établi en laboratoire de sécurité biologique de niveau 3 un modèle expérimental de macaques (*macaca fascicularis*), et avons mené des expériences sur ce modèle fiable de primate non-humain (Lasmezas et al, 1996, Nature 381, 743-744;. Herzog et al., 2005, Journal of virology 79, 14339-14345; Comoy et al, 2008, PLoS One 3, e3017). Nous décrivons ici une nouvelle maladie neurologique mortelle présentant des périodes d'incubation plus ou moins prolongées se développant chez plusieurs macaques exposés à des produits sanguins infectieux provenant de primates ou d'humains.

### Matériaux et méthodes

### Animaux expérimentaux

Des singes mâles de 2-5 ans élevés nés en captivité ont été fournis par Noveprim (Maurice). L'absence d'agents pathogènes primates courants avant l'importation a été vérifiée chez ces singes, et les singes ont été manipulés conformément aux directives nationales. Les animaux ont été maintenus dans un établissement de soins pour animaux de niveau 3. Des examens cliniques ont été effectués régulièrement. Les tissus ont été fixés dans du formol à 4% ou le fixateur de Carnoy pour un examen histologique.

### Tissus et échantillons de sang.

Les échantillons de cerveau (cortex frontal) et de sang de patients atteints de la vMCJ ont été utilisés (patients britanniques # A, B, C, D et E). Deux inoculums de cerveau de primate atteint d'ESB ont été utilisés : un mélange (échantillon # F) du cortex frontal et du cervelet d'un primate inoculé par voie intracérébrale (Lasmezas et al., 1996, Nature 381, 743-744), ou le cortex occipital (échantillon # G) d'un primate exposé par voie orale avec un cerveau de bovin infecté par l'ESB (Lasmezas et al., 2005, Lancet 365, 781-783). Le surnageant correspondant a été obtenu par centrifugation à 1500 g pendant 10 minutes après sonication extensive. Des dilutions en série d'homogénat de cerveau à 10% dans du glucose 5% ont été inoculées par voie intracérébrale (IC), par voie intraveineuse (IV) et / ou dans les amygdales rétro pharyngiennes (ITO).
Des échantillons du sang des primates ont été prélevés dans du citrate de sodium et séparés par centrifugation à 2000 g pendant 13 minutes. Pour les inoculations de primates # 19 à 23, des volumes égaux de sang de primates #14 à #18 ont été prélevés lors de l'apparition des signes cliniques, mis en commun, déleucocytés (Filtre LST2, Maco Pharma, <5x10³ leucocytes/ml après filtration) puis fractionnés. Les globules rouges déleucocytés (CGR-DL) ont été remis en suspension dans leur plasma dans des proportions correspondant à celles utilisées pour la transfusion en pédiatrie. Le CGR-DL a été filtré sur un filtre P-CAPT™ (Maco Pharma, France) selon les recommandations du fabricant.

### Neuropathologie et immunochimie.

La neuropathologie et la détection immunochimique de la protéine prion protéinase-résistante (PrPres) a été réalisée sur des coupes de cerveau, comme décrit précédemment en utilisant l'anticorps 3F4 (Comoy et al., 2008). L'immunohistochimie a également été effectuée sans l'étape de protéolyse.

### Analyse de la PrP.

La PrP a été purifiée selon le protocole de purification TeSeE (Bio-Rad), en présence ou en l'absence de protéinase K. Des d'échantillons purifiés ont été traités pour une analyse par Western blot comme décrit précédemment (Comoy et al., 2008), en utilisant l'anticorps 3F4.

Séquençage en profondeur (recherche du génome des agents pathogènes). L'ARN a été extrait d'échantillons du système nerveux central (Qiagen kit). Un séquençage à haut débit a été effectué comme décrit précédemment (Cheval et al., 2011, J Clin Microbiol 49, 3268-3275).

### Analyse par IRM.

L'Imagerie par résonance magnétique (IRM) a été réalisée sur un système horizontal de Teslas 7. Le positionnement et le suivi des animaux ont été effectués comme décrit précédemment (Valette et al., 2006, J Magn Reson Imaging 23,408-412), en utilisant un équipement dédié de sorte à maintenir le niveau 3 de confinement. Des images pondérées Coronal T2 ont été acquises en utilisant une séquence spin-écho rapide (TE / TR = 60/4000, 450x450µmin en résolution plane, épaisseur de coupe de 1 mm, 40 tranches, temps d'acquisition de 14 min).

### Résultats

Onze animaux (#1, #2, #9-#12 et #14-#18) ont été inoculés avec du tissu cérébral infecté par l'ESB ou la vMCJ pour fournir des animaux donneurs de sang pour les études (tableau 1). Deux animaux (#1 et #2) ont été inoculés avec des tissus cérébraux de patients vMCJ humains par voie intracérébrale (IC) ou intra-amygdalienne (ITO), et neuf animaux (#9-#12 et #14-#18) ont été inoculés par voie intraveineuse (IV) avec les tissus de cerveau de singes ESB. Tous ont développé un schéma classique de vMCJ ou d'ESB après des périodes d'incubation de 2 à 4 ans, dans la plupart des cas, proportionnel à la dose initiale infectieuse, avec les symptômes habituels neurologiques (agressivité et hyperesthésie suivie par une ataxie cérébelleuse, des troubles de locomotion, et des tremblements). Une spongiose et des dépôts de PrPres ont été observés dans les zones du cerveau principal, ainsi qu'une accumulation de PrPres dans les organes lymphoïdes.

Les animaux receveurs (#3-4, #5-8, #13, #19-23) ont été soit inoculés IC soit transfusés, avec des fractions du sang des primates donneurs, prélevés au stade terminal de la maladie, ou avec du sang provenant de quatre patients humains vMCJ. Deux transfusions (à 7 jours d'intervalle), totalisant 40 ml de sang total du primate #1 a induit la vMCJ classique chez le primate # 3 cinq années après l'inoculation.

Parmi les autres receveurs, 8 animaux (dont 2 encore en vie) ont développé un syndrome neurologique qui diffère des maladies classiques vMCJ ou ESB chez cette espèce. Après des périodes d'incubation allant de 5,5 à 12 ans, quatre animaux inoculés avec des produits sanguins d'humains (#5 et #7) ou de primates (# 4 et # 13), ont commencé une longue phase clinique (2 à 6 mois) avec pour conséquence une imprécision des gestes fins au niveau des membres supérieurs (les animaux n'arrivent pas à attraper de petits raisins secs), et ont graduellement cessé d'utiliser leurs mains pour attraper la nourriture uniquement avec leur bouche. Une ataxie des membres inférieurs est apparue après plusieurs semaines, suivie par une parésie proximale progressive des membres supérieurs et une atrophie des muscles des épaules. Une atteinte des nerfs crâniens est également soupçonnée en présence d'une ouverture permanente de la bouche, des bâillements fréquents, et des mouvements anormaux de la langue. Aucun des animaux n'a montré l'une des caractéristiques de la vMCJ / ESB classique: des symptômes d'agressivité, une ataxie cérébelleuse, ou des tremblements. Deux autres primates (# 6 et # 8) inoculés avec des produits sanguins humains (globules blancs + / - plasma) et encore en vie au moment de la rédaction montrent des signes cliniques atypiques semblables.

Les quatre animaux morts (# 4, #5, #7, et #13) présentaient très peu de lésions cérébrales: le cortex et les noyaux gris centraux ont été pratiquement épargnés et les cellules granulaires du cervelet modérément raréfiées. Cependant, ils ont tous montré à des degrés divers des caractéristiques neuropathologiques suivantes : des lésions nécrotiques bilatérales des cornes antérieures de la moelle épinière cervicale basse (figure 1A) s'étendant dans certains cas jusqu'à C4 correspondent à une perte de substance fondamentale remplie avec les macrophages et les cellules gliales, et quelques neurones persistants (figure 1B). Aucun infiltrat de lymphocytes n'était présent. Le même type de lésions a été observé de façon bilatérale dans le tronc cérébral (figure 1C) : cette lésion centrée sur les noyaux spinaux du nerf trijumeau, s'étend dans certains cas aux pédoncules inférieurs du cervelet. Une dégénérescence wallérienne des cordons vertébrales postérieurs (principalement gracilis, figure 1A) a également été observée tout au long de la moelle épinière et sur une partie des tractus optiques (données non présentées).

Les techniques classiques ont été utilisées pour détecter la PrPres dans le système nerveux central (SNC): l'immunohistochimie, l'ELISA (Bio-Rad TeSeE), la purification par SAF, la précipitation NaPTA, combinées avec différents anticorps dirigés contre diverses parties de la protéine PrP (FAS-32, 3F4 , SAF-60, Pri-917 reconnaissant des épitopes 59-89, 109-112, 157-161 et 216-231, respectivement) (Feraudet et al., 2005, J Biol Chem 280, 11247-11258). Aucune de ces méthodes n'a été capable de détecter l'accumulation de PrPres dans n'importe quelle partie du système nerveux central (figure 2A), ou dans les follicules de tout organe lymphoïde (données non présentées). Lorsque le protocole de purification par SAF a été appliqué en l'absence de la protéinase K, des quantités en moyenne plus élevées que chez les animaux témoins de la PrP ont été détectées dans le cerveau des cas atypiques (figure 2B). en l'absence de traitement protéolytique, un marquage spécifique de la PrP a été observée dans la moelle épinière cervicale de ces primates «atypiques», concentrée dans la substance gélatineuse de la corne postérieure (figure 2C et 2D), tandis que les primates contrôles sains ont montré, le cas échéant, un marquage plus faible de cette zone. Cette zone correspond au marquage PrP le plus intense observé dans la moelle épinière de primates infectés par vMCJ (figure 2E et 2F).

Dans une étude indépendante pour évaluer un dispositif de réduction des prions contenant un matériau capable de se lier spécifiquement à la PrP (filtre P-CAPT™), des primates "donneurs" (#14 à #18) ont été injectés IV. avec de grandes doses de cerveau clarifié à partir d'un des primates «typique» ESB (primate #G), leur sang a été prélevé à l'apparition de leurs signes cliniques d'ESB et les concentrés de globules rouges (CGR) ont été resuspendus dans le plasma autologue. À 30 et 31 mois post-transfusion, les deux receveurs contrôles (#19 et #20) transfusés avec ces CGR déleucocytés ont développés des signes de la maladie atypique, alors que le même inoculum transmis à travers le dispositif de filtration P-CAPT™ n'a pas, au moment d'écrire ces lignes (40 mois post-transfusion), induit la maladie dans aucun des trois animaux receveurs (#21 à # 23).

Les six animaux qui sont morts avec le syndrome neurologique atypique décrit ici ont été inclus dans des protocoles expérimentaux de novembre 1998 à avril 2011. Quatre-vingt-cinq autres primates, inoculés par les mêmes voies (IC ou IV) avec des échantillons de cerveau ou de produits sanguins infectés (ou potentiellement infecté) à l'ESB / vMCJ ou d'autres souches de prion liées aux animaux, ont été co-hébergés durant cette période: 33 animaux ont développé une maladie à prions classiques alors que 52 animaux sont encore vivants et asymptomatiques.

Aucun des animaux atteints de la maladie atypique n'a montré de troubles vasculaires ou du métabolisme, et les valeurs en vitamine (B1, B9 et B12) et en oligo-éléments (zinc, cuivre, plomb, magnésium) étaient dans les limites normales. Une origine auto-immune est hautement improbable en l'absence d'auto-anticorps contre les protéines du système nerveux central (données non présentées), des valeurs normales des paramètres céphalo-rachidien et des infiltrats lymphocytaires. La sérologies de ces six animaux est négative pour la rougeole, la varicelle, l'herpès, le SIV, STLV, l'entérovirus, le papovavirus, ou la syphilis.

| Animal | Donneur | Incoulum | Quantité | Voie | Période d'incubation (mois) | Phase clinique (mois) | Profil pathologique¹ |
|---|---|---|---|---|---|---|---|
| #1 | Patient UK vMCJ #A | cerveau | 40 mg | IC | 32 | 4.5 | vMCJ |
| #2 | Patient UK vMCJ #A | Cerveau | 8 mg | ITO | 43 | 1 | vMCJ |
| #3 | Patient UK vMCJ #A | Sang total (ST²) | 10+30ml | IV | 62 | 4.5 | vMCJ |
| #4 | Patient UK vMCJ #A | Buffy coat (ST²) | 0.5 ml | IC | 88 | 4 | atypique |
| #5 | Patient UK vMCJ #A | Buffy coat | 0.5 ml | IC | 107 | 4.5 | atypique |
| #6 | Patient UK vMCJ #A | Buffy coat | 0.5 ml | IC | 125 | >6 | *Atypique ?* |
| #7 | Patient UK vMCJ #A | Plasma+ Buffy coat | 20 ml (eq 40 ml ST³) | IV | 65.5 | 6 | Atypique |
| #8 | Patient UK vMCJ #A | Plasma+ Buffy coat | 7.5 ml (eq 15 ml ST³) | IV | 131 | >6 | *Atypique ?* |
| #9 | Primate BSE #F | Cerveau | 40 mg | IV | 21.5 | 4 | BSE |
| #10 | Primate BSE #F | Cerveau | 4 mg | IV | 36 | 2 | BSE |
| #11 | Primate BSE #F | Cerveau | 0.4 mg | IV | 30 | 3 | BSE |
| #12 | Primate BSE #F | Cerveau | 0.04 mg | IV | 47 | 4 | BSE |
| #13 | Primate BSE #F | Sang total (ST²) | 40 ml | IV | 141 | 5 | Atypique |
| #14 | Primate BSE #G | Cerveau (Surnageant HS) | 100 mg | IV | 35 | 9 | BSE |
| #15 | Primate BSE #G | Cerveau (Surnageant HS) | 100 mg | IV | 33 | 2.5 | BSE |
| #16 | Primate BSE #G | Cerveau (Surnageant HS) | 10 mg | IV | 45.5 | 12 | BSE |
| #17 | Primate BSE #G | Cerveau (Surnageant HS) | 10 mg | IV | 34.5 | 3 | BSE |
| #18 | Primate BSE #G | Cerveau (Surnageant HS) | 10 mg | IV | 42.5 | 9 | BSE |
| #19 | Primate BSE #14-18 | RBC-DL | 27 ml (eq 40 ml ST³) | IV | 30 | 2.5 | Atypique |
| #20 | Primate BSE #14-18 | RBC-DL | 27 ml (eq 40 ml ST³) | IV | 30 | 2 | Atypique |
| #21 | Primate BSE #14-18 | RBC-DL filtré | 27 ml (eq 40 ml ST³) | IV | >40 | | asymptomatique |
| #22 | Primate BSE #14-18 | RBC-DL filtré | 27 ml (eq 40 ml ST³) | IV | >40 | | asymptomatique |
| #23 | Primate BSE #14-18 | RBC-DL filtré | 27 ml (eq 40 ml ST³) | IV | >40 | | asymptomatique |

| | | | | | | | |
|---|---|---|---|---|---|---|---|
| 1 les profils BSE ou vMCJ sont indiqués selon l'inoculum initial, mais ils étaient cliniquement, biochimiquement et au niveau des lésions indiscernables. 2 ST :Stade terminal de la maladie. 3 Chaque primate a été transfusé avec 17 ml de concentré de globules rouges appauvri en leucocytes (CGR-DL) correspondant à 40 ml de sang total, et complété avec 10 ml de plasma déleucocyté correspondant. | | | | | | | |

### Discussion

Nous avons confirmé que la vMCJ pouvait être transmise par transfusion de sang total à des macaques cynomolgus, établissant ainsi la pertinence de ce modèle expérimental pour évaluer le risque d'infection sanguine de la vMCJ chez l'homme. Basé sur le poids d'un singe adulte, 40 ml de sang correspond à une poche de 250 ml de sang pour l'homme. Considérant la période d'incubation observée, le niveau d'infectiosité du sang serait de 7-8 log inférieur à l'infectiosité du cerveau, en accord avec la conclusion de 1-10 LD50/ml de sang dans d'autres modèles expérimentaux (Brown et al, 1998, Transfusion 38, 810-816;. Hunter et al, 2002, J Gen Virol 83, 2897-2905.; Gregori et al, 2006, Lancet 368,2226-2230).

Contrairement à l'un des macaques, huit macaques cynomolgus inoculés avec des produits sanguins provenant de primates ou d'humains infectés au vMCJ ou à l'ESB, ont développé un syndrome d'origine myélopathique auparavant jamais observés ou signalés chez les humains ou chez des primates non humains. Cette maladie est caractérisée par une phase subaiguë clinique de plusieurs mois. Des études de transmission à des primates ou à des rongeurs initiées il y a un an sont jusqu'ici négatives, ce qui est trop tôt pour conclure à l'égard d'une étiologie prion, mais tend à éliminer la considération de toute étiologie conventionnelle pathogène.

Les observations cliniques sont étayées par des études neuropathologiques qui montrent que la maladie est limitée au SNC, et aucune infiltration inflammatoire ou inclusion virale n'a été observée. Ces caractéristiques sont compatibles avec un diagnostic de maladie à prions, mais aucune des lésions spécifiques classiques à l'ESB classique telles qu'une spongiose ou une accumulation de la PrPres n'a été identifiée. À ce jour, nous n'avons pas vu de caractéristiques similaires clinico-neuropathologiques chez les primates inoculés IV ou IC par l'agent de l'ESB ou de la vMCJ. Les hypothèses de maladies auto-immunes, métaboliques ou liées à une carence n'ont pas été confirmées. Une contamination infectieuse d'origine non prion peut être présumée, mais les analyses effectuées tendent à infirmer cette hypothèse.

L'absence de neuropathologie typique ou de PrPres n'exclut pas la possibilité d'une forme atypique d'une maladie à prions. Plusieurs publications ont rapporté des cas où une maladie à prions a été dépourvue de ces lésions, ou a montré peu ou pas de PrPres (Lasmezas et al, 1997, Science 275, 402-405;. Bugiani et al, 2000, Microsc Res Tech 50, 10-15; Zou et al, 2010, Ann Neurol68, 162-172). Par ailleurs, une maladie similaire affectant spécifiquement la moelle épinière cervicale a été décrite il y a quelques années dans des souris transgéniques exprimant la PrP tronquée et infectées par la souche de tremblante RML (Flechsig et al., 2000, Neuron 27,399-408), ce qui suggère que des prions peuvent dans certaines conditions provoquer une myélopathie avec une quasi-absence d'accumulation PrPres.

Au cours de nos études, nous avons fait deux observations qui suggèrent une implication de la PrP dans cette maladie neurologique atypique. Tout d'abord, les animaux touchés ont tendance à avoir des quantités plus élevés que chez les contrôles sains de la PrP protéase-sensible dans le SNC, surtout au niveau de la substance gélatineuse, correspondant à la région contenant la quantité la plus élevée de la PrPres dans les patients atteints de sMCJ (Iwasaki et al., 2005, Acta Neuropathol 110, 490-500). Deuxièmement, les concentrés de globules rouges déleucocytés ont été capables d'induire cette nouvelle maladie dans les deux receveurs à 31 et 32 mois, alors que le passage au travers du filtre P-CAPT™ a, jusqu'ici (40 mois post-transfusion), empêché la maladie chez les trois autres receveurs. Une rétention non-spécifique mécanique de l'agent causal par exclusion de taille avec ce filtre est peu probable étant donné que sa porosité est suffisante pour permettre le passage des globules rouges. La résine spécifique incorporée dans ces filtres a une forte affinité pour la protéine prion normale (PrP et PrPc) et anormale (PrPres), réduit de plus de 3 log l'infectiosité de cerveau artificiellement injecté dans les produits sanguins (Gregori et al., 2006, Transfusion 46, 1152-1161) et élimine l'infectiosité du sang apparente endogène chez un modèle de hamster (Gregori et al., 2006, Lancet 368,2226-2230).

Même si nous ne pouvons pas exclure définitivement une autre cause, cette étude suggère qu'une souche de prion atypique puisse avoir été sélectionnée dans nos primates à partir de produits sanguins infectés par l'agent de la vMCJ ou de l'ESB, avec une physiopathologie différente du schéma classique du vMCJ. La PrP semble en cause, mais l'accumulation périphérique et centrale de la PrPres est indétectable, les lésions cérébrales sont absentes, et les images cliniques et lésionnelles sont principalement axées sur la moelle épinière. L'image clinique de cette maladie, qui partage plusieurs similitudes avec des myélopathies notamment la neuromyélite optique ou la sclérose latérale amyotrophique, n'est pas évocateur d'une maladie à prions, et échapperait aux critères diagnostiques classiques correspondants. Les humains pourraient avoir une moindre sensibilité à cette souche, et les cas futurs seraient encore sous incubation.

En conclusion, nous avons décrit un nouveau syndrome neurologique chez les macaques inoculés avec des échantillons vraisemblablement faiblement infectieux du sang et des composants / produits sanguins qui ne serait pas diagnostiqué comme une maladie à prions si elle devait se produire chez les humains. Les donneurs pré-cliniques ou subcliniques pourraient être plus nombreux que chez les individus infectés avec la vMCJ classiques, et le risque de transmission par voie sanguine ne serait pas entièrement empêché par la déleucocytation ou la nanofiltration. Par contre, le filtre P-CAPT™ de Maco Pharma est capable d'éliminer le risque de transmission de ce nouveau syndrome neurologique.

## Revendications

1. Utilisation d'un matériau capable de se lier à la protéine prion normale PrP ou PrPc ou la protéine prion anormale PrPres, cette protéine anormale étant la forme infectieuse de la protéine prion **caractérisée par** sa résistance à la protéinase K, pour éliminer, dans un produit biologique, l'agent étiologique induisant une maladie neurodégénérative autre que les maladies de Creutzfeldt Jakob, ledit agent n'étant pas ladite protéine prion anormale PrPres et ladite maladie neurodégénérative étant induite par la transfusion ou l'injection à un patient d'un produit biologique issu d'un individu infecté par une souche de prion responsable des maladies de Creutzfeldt Jakob et ne présentant pas d'accumulation de PrPres dans le système nerveux central.

2. Utilisation selon la revendication 1, **caractérisée en ce que** le matériau comprend une matrice liée à un groupement fonctionnel.

3. Utilisation selon la revendication 2, **caractérisée en ce que** le groupe fonctionnel est un groupe fonctionnel hydrophile, hydrophobe ou amphiphile tel qu'une amine primaire, une groupe phényle, n-butyle, 2-(diméthylammonio)éthyle, 2-(triméthylammonio)éthyle, diméthylaminoéthyle ou triméthylaminoéthyle.

4. Utilisation selon la revendication 3, **caractérisée en ce que** ledit groupe fonctionnel est le groupe -OCH2-CHOH-CH2-NH2.

5. Utilisation selon l'une des revendications 2 à 4, **caractérisée en ce que** ladite matrice est une matrice comprenant de la silice ou de l'alumine, une matrice polymère réalisée en polyméthacrylate ou en méthacrylate, une matrice Fractogel™, Toyopearl™ or TSK-GEL™.

6. Utilisation selon l'une des revendications 1 à 5, **caractérisée en ce que** le produit biologique est issu d'un individu infecté par une souche de prion, notamment l'agent étiologique responsable des maladies de Creutzfeldt Jakob.

7. Utilisation selon l'une des revendications 1 à 6, **caractérisée en ce que** le produit biologique est du sang ou un composant sanguin.

8. Utilisation selon l'une des revendications 1 à 6, **caractérisée** en que le produit biologique est un produit dérivé du sanguin tel qu'une préparation d'un ou plusieurs facteurs de coagulations.

9. Utilisation selon l'une des revendications 1 à 8, **caractérisée en ce que** le matériau est incorporé dans une colonne chromatographique.

10. Utilisation selon l'une des revendications 1 à 9, **caractérisée en ce que** le matériau est placé dans une unité de filtration comprenant une ou plusieurs couches d'une matière poreuse.

11. Utilisation selon la revendication 10, **caractérisée en ce que** le matériau est placé entre deux couches de non-tissés.

12. Utilisation selon l'une des revendications 1 à 11, **caractérisée en ce que** ladite maladie neurodégénérative correspond à une neuropathie non inflammatoire affectant préférentiellement la moelle épinière sans atteint des autres organes.

13. Utilisation selon l'une des revendications 1 à 12, **caractérisée en ce que** la maladie neurodégénérative se **caractérise par** une accumulation de PrP non résistante à la protéase dans la moelle épinière.

## Patentansprüche

1. Verwendung eines Materials, das dazu in der Lage ist, an das normale Prion-Protein PrP oder PrPc oder das anormale Prion-Protein PrPres zu binden, wobei dieses anormale Protein die infektiöse Form des Prion-Proteins ist, **gekennzeichnet durch** seinen Widerstand gegen die Proteinase K, um, in einem biologischen Produkt, das ätiologische Mittel zu beseitigen, das eine neurodegenerative Erkrankung außer den Creutzfeldt-Jakob-Erkrankungen induziert, wobei das Mittel nicht das anormale Prion-Protein PrPres ist und die neurodegenerative Erkrankung durch die Transfusion oder die Injektion eines biologischen Produkts in einen Patienten induziert wird, das von einem Individuum stammt, das mit einem Prionstamm infiziert ist, der für die Creutzfeldt-Jakob-Erkrankungen verantwortlich ist und keine Ansammlung von PrPres im zentralen Nervensystem aufweist.

2. Verwendung nach Anspruch 1, **dadurch gekennzeichnet, dass** das Material eine Matrix umfasst, die an eine funktionelle Gruppierung gebunden ist.

3. Verwendung nach Anspruch 2, **dadurch gekennzeichnet, dass** die funktionelle Gruppe eine hydrophile, hydrophobe oder amphiphile funktionelle Gruppe ist, wie z. B. ein primäres Amin, eine Phenylgruppe, n-Butyl, 2-(Dimethylammonio)ethyl, 2-(Trimethylammonio)ethyl, Dimethylaminoethyl oder Trimethylaminoethyl.

4. Verwendung nach Anspruch 3, **dadurch gekennzeichnet, dass** die funktionelle Gruppe die Gruppe -OCH2-CHOH-CH2-NH2 ist.

5. Verwendung nach einem der Ansprüche 2 bis 4, **dadurch gekennzeichnet, dass** die Matrix eine Matrix ist, umfassend Siliciumdioxid oder Aluminiumoxid, eine Polymermatrix, hergestellt aus Polymethacrylat oder aus Methacrylat, eine Fractogel™-, Toyopearl™- oder TSK-GEL™-Matrix.

6. Verwendung nach einem der Ansprüche 1 bis 5, **dadurch gekennzeichnet, dass** das biologische Produkt von einem Individuum stammt, das mit einem Prionstamm infiziert ist, insbesondere dem ätiologischen Mittel, das für die Creutzfeldt-Jakob-Erkrankungen verantwortlich ist.

7. Verwendung nach einem der Ansprüche 1 bis 6, **dadurch gekennzeichnet, dass** das biologische Produkt Blut oder ein Blutbestandteil ist.

8. Verwendung nach einem der Ansprüche 1 bis 6, **dadurch gekennzeichnet, dass** das biologische Produkt ein Produkt ist, das vom Blutprodukt abgeleitet ist, wie z. B. ein Präparat mit einem oder mehreren Gerinnungsfaktoren.

9. Verwendung nach einem der Ansprüche 1 bis 8, **dadurch gekennzeichnet, dass** das Material in eine chromatographische Säule integriert ist.

10. Verwendung nach einem der Ansprüche 1 bis 9, **dadurch gekennzeichnet, dass** das Material in einer Filtereinheit platziert ist, umfassend eine oder mehrere Schichten eines porösen Materials.

11. Verwendung nach Anspruch 10, **dadurch gekennzeichnet, dass** das Material zwischen zwei Vliesschichten platziert ist.

12. Verwendung nach einem der Ansprüche 1 bis 11, **dadurch gekennzeichnet, dass** die neurodegenerative Erkrankung einer nicht entzündlichen Neuropathie entspricht, die vorzugsweise das Rückenmark betrifft, ohne anderen Organe zu befallen.

13. Verwendung nach einem der Ansprüche 1 bis 12, **dadurch gekennzeichnet, dass** die neurodegenerative Erkrankung durch eine Ansammlung von PrP gekennzeichnet ist, die nicht gegen die Protease im Rückenmark resistent ist.

## Claims

1. Use of a material capable of binding with normal prion protein PrP or PrPc or abnormal prior protein PrPres, this abnormal protein being the infectious form of the prion protein **characterised by** proteinase K resistance, to remove, in a biological product, the aetiological agent inducing a neurodegenerative disease other than Creutzfeldt-Jakob diseases, said agent not being said abnormal prior protein PrPres and said neurodegenerative disease being induced by transfusion or injection to a patient of a biological product originating from a subject infected with a prion strain responsible for Creutzfeldt-Jakob diseases and not exhibiting PrPres accumulation in the central nervous system.

2. Use according to claim 1, **characterised in that** the material comprises a matrix bound to a functional group.

3. Use according to claim 2, **characterised in that** the functional group is a hydrophilic, hydrophobic or amphiphilic functional group such as a primary amine, a phenyl, n-butyl, 2-(dimethylammonio)ethyl, 2-(trimethylammonio)ethyl, dimethylaminoethyl or trimethylaminoethyl group.

4. Use according to claim 3, **characterised in that** said functional group is the group -OCH2-CHOH-CH2-NH2.

5. Use according to one of claims 2 to 4, **characterised in that** said matrix is a matrix comprising silica or alumina, a polymer matrix made of polymethacrylate or methacrylate, a Fractogel™, Toyopearl™ or TSK-GEL™ matrix.

6. Use according to one of claims 1 to 5, **characterised in that** the biological product originates from a subject infected with a prion strain, particularly the aetiological agent responsible for Creutzfeldt-Jakob diseases.

7. Use according to one of claims 1 to 6, **characterised in that** the biological product is blood or a blood component.

8. Use according to one of claims 1 to 6, **characterised in that** the biological product is a blood-derived product such as a preparation of one or a plurality of clotting factors.

9. Use according to one of claims 1 to 8, **characterised in that** the material is incorporated in a chromatographic column.

10. Use according to one of claims 1 to 9, **characterised in that** the material is placed in a filtration unit comprising one or a plurality of layers of a porous material.

11. Use according to claim 10, **characterised in that** the material is placed between two layers of non-woven materials.

12. Use according to one of claims 1 to 11, **characterised in that** said neurodegenerative disease corresponds to a non-inflammatory neuropathy preferentially affecting the spinal cord with no involvement of other organs.

13. Use according to one of claims 1 to 12, **characterised in that** the neurodegenerative disease is **characterised by** non-protease-resistant PrP accumulation in the spinal cord.
